# EUROPEAN PATENT SPECIFICATION

(11) **EP 3 383 856 B1**
(45) Date of publication and mention of the grant of the patent: **01.01.2020**
(21) Application number: 16801537.8
(22) Date of filing: 28.11.2016
(51) Int. Cl.: C07D 301/12, C07D 301/32

(54) **PROCESS FOR THE EPOXIDATION OF PROPENE**
VERFAHREN ZUR EPOXIDIERUNG VON PROPEN
PROCEDE D'EPOXYDATION DE PROPENE

(30) Priority: 02.12.2015 EP 15197582
(43) Date of publication of application: 10.10.2018
(73) Proprietor: Evonik Operations GmbH, 45128 Essen (DE); thyssenkrupp Industrial Solutions AG, 45143 Essen (DE)
(72) Inventor: BERNHARD, Maik, 60596 Frankfurt (DE); DIETZ, Hans-Christian, 65795 Hattersheim (DE); SCHMIDT, Franz, 60386 Frankfurt (DE); PASCALY, Matthias, 60599 Frankfurt (DE); WÖLL, Wolfgang, 63477 Maintal (DE)
(74) Representative: Thiele, Georg Friedrich
(86) International application number: PCT/EP2016/079049
(87) International publication number: WO 2017/093204

(56) References cited:
- WO-A1-2004/048354
- WO-A1-2014/193889
- US-A1- 2004 006 239
- US-A1- 2011 245 519

## Description

### Field of the invention

The present invention relates to a process for the epoxidation of propene with hydrogen peroxide in the presence of a titanium silicalite fixed bed catalyst.

### Background of the invention

The epoxidation of propene with hydrogen peroxide in the presence of a titanium silicalite catalyst is known from EP 0 100 119 A1. The reaction of propene with hydrogen peroxide in the presence of a titanium zeolite catalyst is usually carried out in a methanol solvent to achieve high reaction rates and product selectivity. The crude propene oxide provided by this reaction contains residual methanol solvent and volatile by-products formed in the reaction and the work-up, in particular the aldehydes formaldehyde, acetaldehyde and propionaldehyde, and their acetals with methanol and 1,2-propanediol, as well as methyl formate.

Most of the propene oxide is further reacted to polyether polyols which are used as monomers for making polyurethanes. For this purpose, the propene oxide has to be of high purity, containing less than 200 ppm water, less than 100 ppm methanol and less than 50 ppm acetaldehyde. Methanol and acetaldehyde are difficult to remove from propene oxide by simple distillation. In mixtures containing more than 98 mol-% propene oxide these compounds have essentially the same volatility than propene oxide. Therefore distillative purification to low levels of methanol and acetaldehyde is not feasible via conventional distillation.

EP 0 004 019 A2 discloses a method for purifying an epoxide or a mixture comprising an epoxide containing carbonyl compounds, where the epoxide is fed to the middle section of a distillation column and a compound containing one or several unsubstituted NH₂ groups is fed above the feeding point fro the epoxide. 1,2-diaminoalkanes with 2 to 5 carbon atoms are disclosed as particularly suitable and example 6 describes the purification of a benzene solution of trans-2,3-butene oxide using ethylene diamine as the compound containing one or several unsubstituted NH2 groups.

WO 2004/048355 discloses a method for removing methanol and acetaldehyde from a crude propene oxide in a single distillation column by an extractive distillation where a compound containing an unsubstituted NH₂ group and capable of reacting with acetaldehyde at the conditions of distillation is additionally fed at or above the feeding point of the crude propene oxide. An aqueous hydrazine solution is preferably used as the additionally fed compound. Water is particularly preferred as the extraction solvent. The method provides propene oxide of high purity suitable for making polyether polyols.

WO 2004/048355 contains no teaching or suggestion that 1,2-diaminoalkanes or 1,3-diaminoalkanes are capable of reacting with acetaldehyde at the conditions of distillation when water is used as the extraction solvent. Aminals and imines of aliphatic aldehydes with alkyl amines are known to be unstable and rapidly hydrolyze with water to form the aldehyde and amine. The same holds for imidazolidine and its alkyl derivatives. Therefore, based on the common knowledge on the instability of the reaction products of 1,2-diaminoalkanes and 1,3-diaminoalkanes with acetaldehyde towards water and their rapid hydrolysis with water, a person skilled in the art would not expect these compounds to be useful in the process of WO 2004/048355 when using water as the extraction solvent.

WO 2004/029032 teaches to carry out the epoxidation of olefins with hydrogen peroxide in the presence of a titanium-containing zeolite catalyst in an aqueous reaction mixture comprising less than 100 wppm alkali metals, earth alkali metals and strong bases or cations of such bases having a pK_{B} of less than 4.5 and at least 100 wppm weak bases or cations of such bases having a pK_{B} of at least 4.5. Limiting the amounts of alkali metals, earth alkali metals and strong bases reduces or prevents long term deactivation of the catalyst, whereas the presence of the weak base improves the selectivity for the epoxide without affecting the long-term activity of the catalyst.

WO 2004/048354 teaches recovery of a solvent stream from the reaction mixture of an epoxidation of an olefin with hydrogen peroxide in the presence of a titanium-containing zeolite catalyst, where the recovered solvent stream is treated to contain less than 50 wppm nitrogen in the form of organic nitrogen compounds before it is recycled to the epoxidation step, in order to reduce deactivation of the catalyst upon recycling of the solvent. The solvent treatment is preferably an acid treatment with a mineral acid, a carboxylic acid or an acidic ion exchanger.

The examples of WO 2004/048354 describe a process for the epoxidation of propene comprising the steps:
reacting propene with hydrogen peroxide in the presence of a methanol solvent and a titanium zeolite epoxidation catalyst to provide a reaction mixture,
separating a crude propene oxide, comprising propene oxide and methanol, and a solvent mixture, comprising methanol, water and hydrogen peroxide, from the reaction mixture,
subjecting the crude propene oxide to an extractive distillation in an extractive distillation column, using a 1.5 % by weight aqueous hydrazine solution as extraction solvent to provide purified propene oxide as an overhead product and a bottoms product comprising water and methanol, combining the solvent mixture that was separated from the reaction mixture with the bottoms product from the extractive distillation,
subjecting the combined mixture to catalytic hydrogenation,
separating the hydrogenated mixture in a distillation stage to provide a recovered methanol as an overhead product, adding acetic acid to the distillation, and
recycling the recovered methanol to the epoxidation reaction.

### Summary of the invention

It has now been found that, contrary to what one would expect from the known properties of imines and imidazolidines, the removal of methanol and acetaldehyde from crude propene oxide taught in WO 2004/048355 can be successfully carried out using a combination of water as extraction solvent and of a diaminoalkane having from 2 to 6 carbon atoms as compound reacting with acetaldehyde. It has also been found that the use of such a combination in the process of WO 2004/048354 reduces the content of alkylamines in the hydrogenated mixture compared to the use of an aqueous solution of hydrazine. This reduces the amount of strongly basic alkyl amines recycled with the methanol to the epoxidation step which improves the long-term stability of the epoxidation catalyst and reduces the need for further removal of nitrogen compounds from the recycle methanol by additional means, such as additional treatment with an acidic ion exchanger. It also allows recycle of methanol with addition of less acid to the distillation for methanol recovery, which reduces acid corrosion in the bottom section and the reboiler of the distillation column for methanol recovery.

Subject of the invention is therefore a process for the epoxidation of propene comprising the steps
a) reacting propene with hydrogen peroxide in the presence of a methanol solvent and a titanium zeolite epoxidation catalyst to provide a reaction mixture,
b) separating a crude propene oxide, comprising from 15 to 97 % by weight propene oxide, from 2 to 84 % by weight methanol and acetaldehyde, and a solvent mixture, comprising methanol, water and peroxides, from the reaction mixture of step a),
c) subjecting the crude propene oxide separated in step b) to an extractive distillation in an extractive distillation column, using water as extraction solvent to provide purified propene oxide as an overhead product and a bottoms product comprising water and methanol,
d) subjecting the solvent mixture separated in step b) and the bottoms product of step c) to catalytic hydrogenation to form a hydrogenated solvent mixture,
e) separating the hydrogenated solvent mixture in at least one distillation stage to provide a recovered methanol as an overhead product, adding an acid to the hydrogenated solvent mixture or to at least one distillation stage, and
f) recycling the recovered methanol of step e) to step a), wherein in step c) a reactive compound selected from diaminoalkanes having 2 to 6 carbon atoms is added to the extractive distillation either with a feed stream to the extractive distillation column or directly to the extractive distillation column at a feed point above the feed point for the crude propene oxide.

### Detailed description of the invention

The process of the invention comprises a step a) of reacting propene with hydrogen peroxide in the presence of a methanol solvent and a titanium zeolite epoxidation catalyst which provides a reaction mixture. The propene may contain propane, preferably with a molar ratio of propane to propene of from 0.001 to 0.15 and more preferably of from 0.08 to 0.12. Hydrogen peroxide can be used as an aqueous solution, preferably containing from 30 to 75 % by weight hydrogen peroxide and most preferably from 40 to 70 % by weight. Preferably, an aqueous hydrogen peroxide solution made by an anthraquinone process is used. The molar ratio of propene to hydrogen peroxide is preferably from 1.1:1 to 30:1, more preferably 2:1 to 10:1 and most preferably 3:1 to 5:1. The methanol solvent can be a technical grade methanol, a solvent stream recovered in the work-up of the epoxidation reaction mixture or a mixture of both. The methanol may comprise other solvents in minor amounts, such as ethanol, with the amount of such other solvents preferably being less than 2 % by weight. The methanol solvent is preferably used in a weight ratio of 0.5 to 20 relative to the amount of aqueous hydrogen peroxide solution.

The epoxidation reaction of step a) is preferably carried out at a pressure that is higher than the vapor pressure of propene at the reaction temperature in order to maintain the olefin dissolved in the methanol solvent or present as a separate liquid phase. The pressure is preferably from 1.9 to 5.0 MPa, more preferably 2.1 to 3.6 MPa and most preferably 2.4 to 2.8 MPa. Propene is preferably used in an excess sufficient to maintain an additional liquid phase rich in propene throughout the epoxidation reaction. Using an excess of propene at a high pressure provides high reaction rate and hydrogen peroxide conversion and at the same time high selectivity for propene oxide. The reaction is preferably carried out at a temperature of 20 to 80°C, more preferably of 25 to 60°C.

The epoxidation reaction of step a) is carried out in the presence of a titanium zeolite epoxidation catalyst. Suitable titanium zeolites contain titanium atoms on silicon lattice positions. Preferably, a titanium silicalite catalyst is used, preferably with an MFI or MEL crystal structure. Most preferably a titanium silicalite 1 catalyst with MFI structure as known from EP 0 100 119 A1, is used. The titanium silicalite catalyst is preferably employed as a shaped catalyst in the form of granules, extrudates or shaped bodies. Shaping can be carried out by any method known from the prior art for shaping a titanium silicalite powder. Preferably, the shaped titanium silicalite catalyst is prepared by an extrusion process where a kneadable mass of a titanium silicalite powder, a liquid, a binder or binder precursor, and optionally processing additives is pressed through a die, the formed strands are cut, dried to green bodies and calcined to form extrudates. The shaped titanium silicalite catalyst is therefore preferably in the form of extrudates, preferably having a cylindrical shape, where the edges at the end of the cylinders may optionally be rounded. The cylinders of such shaped catalyst preferably have a diameter of from 1 to 5 mm and a length of from 2 to 7 mm. The extrudates preferably comprise a silica binder. Suitable binder precursors for a silica binder that can be used in an extrusion process are fumed or precipitated silicas, silica sols, silicone resins or silicone oils, such as polydimethylsiloxanes, and tetraalkoxysilanes, such as tetraethoxysilane. Shaping can be carried out with a calcined titanium silicalite powder or with an uncalcined titanium silicalite powder still containing template molecules within the zeolite framework. When shaping is carried out with an uncalcined titanium silicalite powder, the catalyst is calcined after shaping in order to remove the template from the zeolite framework. The amount of catalyst employed may be varied within wide limits and is preferably chosen so that a hydrogen peroxide consumption of more than 90%, preferably more than 95%, is achieved within 1 minute to 5 hours under the employed epoxidation reaction conditions.

The epoxidation is preferably carried out in a fixed bed reactor by passing a mixture comprising propene, hydrogen peroxide and methanol over a fixed bed comprising a shaped titanium silicalite catalyst. The fixed bed reactor is preferably equipped with cooling means and cooled with a liquid cooling medium. Preferably, a tube bundle reactor comprising a multitude of parallel reaction tubes and a cooling jacket enclosing the reaction tubes is used. The tube bundle reactor preferably comprises from 5000 to 20000 parallel reaction tubes, more preferably from 7500 to 15000 parallel reaction tubes. The reaction tubes preferably have a circular cross section with an internal diameter of preferably from 2 to 5 cm, more preferably from 2.5 to 4 cm. Preferably all reaction tubes of the tube bundle reactor have the same internal diameter. The reaction tubes preferably have a length of from 5 to 18 m, more preferably from 10 to 15 m. The catalyst fixed bed preferably extends over more than 70 % of the length of the reaction tubes, more preferably over 90 to 98 % of the length of the reaction tubes. Preferably, the temperature distribution along the length of the catalyst fixed bed is adjusted to keep the reaction temperature along 70 to 98 %, preferably along 80 to 95 %, of the length of the catalyst fixed bed within a range of less than 5 °C, preferably within a range of from 0.5 to 3 °C. The epoxidation reaction mixture is preferably passed through the catalyst bed in down flow mode, preferably with a superficial velocity from 1 to 100 m/h, more preferably 5 to 50 m/h, most preferred 5 to 30 m/h. The superficial velocity is defined as the ratio of volume flow rate/cross section of the catalyst bed. Additionally it is preferred to pass the reaction mixture through the catalyst bed with a liquid hourly space velocity (LHSV) from 1 to 20 h⁻¹, preferably 1.3 to 15 h⁻¹. It is particularly preferred to maintain the catalyst bed in a trickle bed state during the epoxidation reaction. Suitable conditions for maintaining the trickle bed state during the epoxidation reaction are disclosed in WO 02/085873 on page 8 line 23 to page 9 line 15. When the olefin is propene, the epoxidation reaction is most preferably carried out with a catalyst fixed bed maintained in a trickle bed state at a pressure close to the vapor pressure of propene at the reaction temperature, using an excess of propene that provides a reaction mixture comprising two liquid phases, a methanol rich phase and a propene rich liquid phase. Two or more fixed bed reactors may be operated in parallel or in series in order to be able to operate the epoxidation process continuously when regenerating the epoxidation catalyst. Regeneration of the epoxidation catalyst can be carried out by calcination, by treatment with a heated gas, preferably an oxygen containing gas or by a solvent wash, preferably by the periodic regeneration described in WO 2005/000827. Different methods of regeneration may also be combined.

In a preferred embodiment, ammonia is added in step a) with a weight ratio of ammonia to the initial amount of hydrogen peroxide of from 0.0001 to 0.003. The ammonia is preferably added to a feed stream to the reaction, preferably to the methanol solvent.

In step b) of the process of the invention, a crude propene oxide, comprising from 15 to 97 % by weight propene oxide, from 2 to 84 % by weight methanol and acetaldehyde, and a solvent mixture, comprising methanol, water and peroxides, are separated from the reaction mixture of step a). Preferably, the reaction mixture of step a) is subjected to a pressure reduction and propene vapor formed by the pressure reduction is recompressed and cooled to recover propene by condensation. The compressed propene vapor is preferably fed to a propene distillation column and separated into an overhead product comprising non-reacted propene and a bottoms product containing compounds having a boiling point higher than propene, such as propene oxide and methanol solvent. The overhead product comprising non-reacted propene can be recycled to the epoxidation reaction. The bottoms product can be combined with the liquid mixture remaining after the pressure reduction. The liquid mixture remaining after the pressure reduction is preferably separated by distillation in a pre-separation column to provide the crude propene oxide as the overhead product and the solvent mixture as the bottoms product. The pre-separation column is preferably operated to provide an overhead product comprising from 20 to 60 % of the methanol contained in the liquid phase remaining after the pressure reduction. The pre-separation column preferably has from 5 to 20 theoretical separation stages in the stripping section and less than 3 theoretical separation stages in a rectifying section and is most preferably operated without reflux and without a rectifying section to minimize the residence time of propene oxide in the pre-separation column. The pre-separation column is preferably operated at a pressure of from 0.16 to 0.3 MPa. Preferably, propene oxide and methanol are condensed from the overhead product of the pre-separation column and propene is stripped from the resulting condensate in a propene stripping column to provide a crude propene oxide as the bottom stream which is essentially free of propene. The crude propene oxide preferably comprises from 4 to 4000 ppm by weight acetaldehyde. The crude propene oxide may also comprise other aldehydes and ketones, such as formaldehyde, propionaldehyde and acetone, as well as acetals of these aldehydes with methanol or 1,2-propanediol, such as dimethoxymethane, 1,1-dimethoxyethane, 1,1-dimethoxypropane, 4-methyl-1,3-dioxolane, 2,4-dimethyl-1,3-dioxolane and 2-ethyl-4-methyl-1,3-dioxolane. The crude propene oxide may also comprise further solvents, such as ethanol or water. Acetaldehyde and formaldehyde are believed to be formed by oxidative cleavage of propene oxide ring-opening products. The crude propene oxide may also comprise further solvents, such as ethanol or water.

In step c) of the process of the invention, the crude propene oxide separated in step b) is subjected to an extractive distillation in an extractive distillation column, using water as extraction solvent, to provide purified propene oxide as an overhead product and a bottoms product comprising water and methanol. A reactive compound selected from diaminoalkanes having from 2 to 6 carbon atoms is added to the extractive distillation either with a feed stream to the extractive distillation column or directly to the extractive distillation column at a feed point above the feed point for the crude propene oxide. The reactive compound preferably contains two amino groups separated by two or three carbon atoms. The reactive compound is preferably selected from 1,2-diaminoethane, 1,2-diaminopropane and 1,3-diaminopropane and is most preferably 1,2-diaminoethane. The amount of reactive compound fed to the distillation column is preferably chosen so that the molar ratio of the reactive compound relative to acetaldehyde is in the range of from 0.5 to 10, more preferably from 3 to 8. The use of such an amount of reactive compound provides effective conversion of carbonyl compounds to high boiling compounds and provides a propene oxide product with a low content of acetaldehyde and other carbonyl compounds. At the same time, by-product formation by reactions of the reactive compound with propene oxide can be kept at a low level.

The extractive distillation is carried out in an extractive distillation column. The extractive distillation column may be a tray column containing discrete trays such as sieve trays or bubble cap trays. The extractive distillation column may also be a packed column and both random packings as well as structured packings, such as metal gauze packings may be used. The extractive distillation column may also combine sections with discrete trays and sections with packings. The extractive distillation column will in general also comprise at least one overhead condenser and at least one column reboiler. The extractive distillation column has at least two feed points, a feed point A for feeding the crude propene oxide in the middle section of the extractive distillation column and a feed point B for feeding the extraction solvent located above feed point A. The feed points define three sections of the extractive distillation column, a stripping section between the column bottoms and feed point A, an extraction section between feed point A and feed point B and a rectifying section between feed point B and the top of the extractive distillation column. Preferably a distillation column is used that has a separation efficiency of 10 to 30 theoretical stages in the stripping section, a separation efficiency of 15 to 40 theoretical stages in the extraction section and a separation efficiency of 20 to 60 theoretical stages in the rectifying section, i.e. feed point B is preferably located from 15 to 40 theoretical separation stages above feed point A and from 20 to 60 theoretical separation stages below the top of the extractive distillation column.

The crude propene oxide is fed to a feed point A of the extractive distillation column and the extraction solvent is fed to a feed point B of the extractive distillation column. The extraction solvent preferably comprises more than 80 % by weight water, more preferably more than 90 % by weight water. Preferably, the extraction solvent comprises no further solvent in addition to water. The extraction solvent is preferably fed in an amount providing a mass ratio of the extraction solvent relative to the amount of methanol contained in the crude propene oxide fed of from 0.01 to 1, more preferably from 0.03 to 0.2. The use of such an amount of extraction solvent provides effective extraction of methanol and a propene oxide product with a low content of methanol and at the same time avoids hydrolysis of propene oxide in the extractive distillation column. The reactive compound may be fed to the extractive distillation column at feed point B, at feed point A or at an additional feed point C between feed points A and B. Preferably, the reactive compound is fed to the extractive distillation column at feed point B admixed with the aqueous extraction solvent.

In a further embodiment of the invention, the crude propene oxide is mixed with an aqueous alkaline solution and the resulting mixture is reacted for 1 to 60 minutes at a temperature of from 20 to 100°C before the mixture is fed to the extractive distillation. The aqueous alkaline solution is preferably an aqueous solution of sodium hydroxide, potassium hydroxide, or sodium carbonate. Most preferred are aqueous sodium hydroxide solutions containing from 0.1 to 56 % by weight sodium hydroxide. The amount of the aqueous alkaline solution is preferably chosen so that the molar ratio of hydroxide ions introduced with the aqueous alkaline solution relative to the amount of methyl formate contained in the crude propene oxide is in the range from 1.1 to 4. Reacting the crude propene oxide with an aqueous alkaline solution converts methyl formate contained in the crude propene oxide by hydrolyzing it to methanol and formate. The purified propene oxide obtained with this embodiment of the invention has a reduced content of methyl formate. Preferably the amount of aqueous alkaline solution is chosen to obtain a purified propene oxide having a content of methyl formate of less than 100 ppm by weight.

In step d) of the process of the invention, the solvent mixture separated in step b) and the bottoms product of step c) are subjected to a catalytic hydrogenation to form a hydrogenated solvent mixture. The solvent mixture separated in step b) and the bottoms product of step c) may be fed separately to the catalytic hydrogenation or may preferably be combined before subjecting them to catalytic hydrogenation.

The catalytic hydrogenation is preferably carried out at a hydrogen partial pressure of from 0.5 to 30 MPa, more preferably of from 1 to 25 MPa and most preferably of from 1 to 5 MPa. The temperature is preferably in the range of from 80 to 180 °C, more preferably from 90 to 150 °C. The catalytic hydrogenation is carried out in the presence of a hydrogenation catalyst, preferably a heterogeneous hydrogenation catalyst. Raney nickel and Raney cobalt may be used as hydrogenation catalyst. Preferably, a supported metal catalyst comprising one or more of metals selected from the group consisting of Ru, Rh, Pd, Pt, Ag, Ir, Fe, Cu, Ni and Co on a catalyst support is used. The metal is preferably platinum, palladium, iridium, ruthenium or nickel and most preferably ruthenium or nickel. The catalyst support can be any solid which is inert and does not deteriorate under the hydrogenation conditions. Suitable as catalyst support are activated carbon, the oxides SiO₂, TiO₂, ZrO₂ and Al₂O₃, and mixed oxides comprising at least two of silicon, aluminum, titanium and zirconium. Activated carbon is preferably used as the catalyst support for the supported metal catalyst. The catalyst support is preferably shaped as spheres, pellets, tablets, granules or extrudates. Preferred are extrudates with a diameter of from 0.5 to 5mm, especially from 1 to 3 mm, and a length of from 1 to 10 mm. The supported metal catalyst preferably comprises from 0.01 to 60 wt.% metal. Supported noble metal catalysts preferably comprise from 0.1 to 5 % metal. Supported nickel and cobalt catalysts preferably comprise from 10 to 60 % metal. The supported metal catalyst may be prepared by methods known in the art, preferably by impregnating the catalyst support with a metal salt followed by reducing the metal salt to the catalytically active metal. Suitable supported metal catalyst are commercially available, for example from Clariant under the NISAT® trade name and from Evonik Industries under the Noblyst® trade name.

The catalytic hydrogenation converts unreacted hydrogen peroxide to water and the by-products 1-hydroperoxy-2-propanol and 2-hydroperoxy-1-propanol of step a) to 1,2-propanediol and prevents by-product formation by peroxide decomposition in subsequent work-up stages. The hydrogenation also converts aldehyde and ketone by-products to the corresponding alcohols. Depending on the reaction conditions, acetals with methanol or 1,2-propanediol may be hydrogenated as well. Feeding both the solvent mixture separated in step b) and the bottoms product of step c) to the catalytic hydrogenation surprisingly provides a low content of volatile alkyl amines, such as trimethylamine, ethyldimethylamine or diethylmethylamine, in the hydrogenated solvent mixture even when ammonia is added in step a). Without wishing to be bound by theory, the inventors believe this to be due to the diaminoalkane and its reaction products with aldehydes being more reactive in reductive amination than ammonia and thus suppressing the formation of volatile alkyl amines by reductive amination of formaldehyde and acetaldehyde with ammonia.

In step e) of the process of the invention, the hydrogenated solvent mixture obtained in step d) is separated in at least one distillation stage to provide a recovered methanol as an overhead product. An acid is added either to the hydrogenated solvent mixture before it is fed to the distillation or to at least one of the distillation stages, preferably to the first distillation stage. When the acid is added to a distillation stage, it is preferably added at a feed point above the feed point for the hydrogenated solvent mixture and below the column top. The acid may also be added to the reflux stream of the distillation column. Most preferably, the hydrogenated solvent mixture is separated in two subsequent distillation stages providing recovered methanol as an overhead product from both stages, feeding the acid to the hydrogenated solvent mixture before it is fed to the first distillation stage. The two distillation stages are preferably operated with a higher pressure in the second stage and overhead product vapor from the second stage is used for heating the bottoms evaporator of the first stage in order to save energy.

The acid is preferably added in an amount providing a content of less than 50 ppm by weight nitrogen in the form of organic nitrogen compounds in the recovered methanol. The acid may be a mineral acid, such as nitric acid, sulfuric acid, hydrochloric acid, phosphoric acid or perchloric acid; a sulfonic acids, such as methane sulfonic acid; or a carboxylic acid, such as formic acid, acetic acid, propionic acid, butyric acid, pentanoic acid, hexanoic acid, heptanoic acid, octanoic acid, nonanoic acid, decanoic acid, undecanoic acid, dodecanoic acid, oxalic acid, malonic acid, succinic acid, glutaric acid, adipic acid, maleic acid or fumaric acid. Preferred are sulfuric acid and phosphoric acid, most preferred is sulfuric acid. The amount of nitrogen in the form of organic nitrogen compounds can be determined as the difference between the total amount of nitrogen and the amount of nitrogen in the form of inorganic nitrogen compounds. The total amount of nitrogen can be determined by the Kjeldahl method as described in DIN 53625. The recovered methanol will usually contain no inorganic compounds other than ammonia and the amount of nitrogen in the form of inorganic nitrogen compounds may therefore be determined by ion chromatography of an acidified sample detecting ammonium ions.

The acid is preferably added in an amount providing an apparent pH of from 1.8 to 5.0, more preferably from 1.9 to 4.0, in the bottoms product remaining after recovery of methanol. The term apparent pH here refers to the value measured at 20 °C with a pH meter equipped with a pH sensitive glass electrode calibrated with aqueous buffer solutions. Maintaining the apparent pH within these ranges provides a recovered methanol with a low content of alkyl amines and at the same time reduces or prevents acid corrosion of the distillation equipment.

Since the use of a diaminoalkane as claimed instead of hydrazine as a reactive compound in step c) reduces the concentration of volatile alkylamines in the hydrogenated solvent mixture obtained in step d), step e) can be carried out with less acid than needed in the process of WO 2004/048354 which uses hydrazine. This reduces acid corrosion of the distillation equipment, as well as the amount of acid needed in step e) and the amount of salts contained in the bottoms product remaining after recovery of methanol.

In step f) of the process of the invention, the recovered methanol of step e) is recycled to step a). The recovered methanol of step e) may be recycled directly to step a). Alternatively, all or a part of the recovered methanol of step e) may be used as an absorbent for absorbing propene from an off-gas stream resulting from step a), step b) or both steps and the recovered methanol loaded with propene is recycled to step a).

In a preferred embodiment, the recovered methanol of step e) is passed through a bed of an acidic ion exchanger before recycling it to step a). Both strongly acidic ion exchangers and weakly acidic ion exchangers may be used. Preferred are strongly acidic ion exchangers containing SO₃H groups and weakly acidic ion exchangers containing COOH groups. The acidic ion exchanger is preferably based on an organic polymer, such as crosslinked polystyrene, or an organic inorganic hybrid polymer, such as a polysiloxane. The acidic ion exchanger may be a gel type solid or a macroporous solid. Preferably, two ion exchanger beds are arranged in parallel to allow regeneration of the ion exchanger without interrupting the methanol treatment.

When the amount of acid added in step e) is not substantially reduced compared to the prior art process of WO 2004/048354, the use of a diaminoalkane as claimed instead of hydrazine as a reactive compound in step c) will reduce the concentration of volatile alkylamines in the recovered methanol. This will lead to an increased time on stream for the ion exchanger used for treating the recovered methanol, i.e. the ion exchangers have to be regenerated less frequently which reduces the consumption of chemicals for their regeneration.

Steps a) to f) of the process of the invention are preferably carried out continuously.

### Examples

### Example 1, reactivity of diamines to propionaldehyde

A mixture of 60 g propene oxide, 35 g methanol and 0.1 g propionaldehyde was placed in a vessel equipped with a reflux condenser and heated to reflux under a nitrogen atmosphere. 5 min after the mixture started to boil a solution of the diamine in 5 g of water was added, the amount of diamine being chosen to provide the molar ratio of diamine to propionaldehyde given in table 1, and after a further 7 min at reflux a sample was withdrawn and analyzed by GC for propionaldehyde conversion. Comparative experiments were carried out with hydrazine, ethanolamine and cyclohexylamine instead of the diamine. Table 1 summarizes the amines and the molar ratios tested and the determined propionaldehyde conversions. The results given in table 1 demonstrate that diaminoalkanes having from 2 to 6 carbon atoms are unexpectedly effective for converting aldehydes to higher boiling products even when substantial amounts of water are present.

**Table 1**

| Added amino compound | Propionaldehyde conversion at molar ratio 1.1 | Propionaldehyde conversion at molar ratio 2.2 |
|---|---|---|
| Hydrazine* | 92 | n.d. |
| 1,2-Diaminoethane | 80 | 87 |
| 1,2-Diaminopropane | 85 | 88 |
| 1,3-Diaminopropane | 84 | 90 |
| 1,6-Diaminohexane | 59 | 71 |
| Ethanolamine* | 44 | n.d. |
| Cyclohexylamine* | 29 | n.d. |

| | | |
|---|---|---|
| *not according to the invention n.d. = not determined | | |

### Example 2, reactivity of 1,2-diaminoethane to acetaldehyde

Example 1 was repeated with 1,2-diaminoethane and hydrazine for comparison, placing a mixture containing 50.1 % by weight propene oxide, 44.7 % by weight methanol, 5.2 % by weight water, 175 ppm acetaldehyde and 60 ppm propionaldehyde in the vessel. For a molar ratio of diamine to (acetaldehyde + propionaldehyde) of 2.3, an acetaldehyde conversion of 88 % and a propionaldehyde conversion of 33 % were observed in the experiment with 1,2-diaminoethane and an acetaldehyde conversion of 85 % and a propionaldehyde conversion of 23 % were observed in the comparative experiment with hydrazine. For a molar ratio of diamine to (acetaldehyde + propionaldehyde) of 4.6, an acetaldehyde conversion of 96 % and a propionaldehyde conversion of 29 % were observed in the experiment with 1,2-diaminoethane and an acetaldehyde conversion of 98 % and a propionaldehyde conversion of 29 % were observed in the comparative experiment with hydrazine. The results demonstrate that 1,2-diaminoethane has a reactivity to acetaldehyde similar to hydrazine even when substantial amounts of water are present.

### Example 3, epoxidation of propene with extractive distillation of crude propene oxide adding hydrazine as reactive compound (comparative)

Propene was epoxidized in a pilot plant reactor in a reaction tube equipped with a cooling jacket. A catalyst fixed bed of 10 liter extruded titanium silicalite catalyst was arranged in the reaction tube. A mixture comprising 40 % by weight of propene, 7.7 % by weight hydrogen peroxide, 3.3 % by weight water, 49 % by weight of methanol and 80 ppm ammonia was fed to the top of the reaction tube and passed through the catalyst fixed bed in trickle mode. The pressure in the reactor was kept at 2.6 MPa by introducing nitrogen. The temperature in the reactor was kept essentially constant at a temperature in the range of from 25 to 60 °C, adjusting the temperature during the epoxidation reaction to maintain an essentially constant conversion of hydrogen peroxide of 97 %.

The reaction mixture exiting the reactor was depressurized to a pressure of 0.25 MPa and the depressurized liquid was fed to a distillation in a pre-separation column to provide an overhead product comprising propene oxide, methanol, residual propene and acetaldehyde and a bottoms product comprising methanol, water and non-reacted hydrogen peroxide. Propene oxide and methanol were condensed from the overhead product of the pre-separation column and propene was stripped from the resulting condensate in a propene stripping column to provide a crude propene oxide as bottom stream comprising 52 % by weight propene oxide, 44 % by weight methanol and 260 ppm acetaldehyde.

The crude propene oxide was fed to stage 42 (counted from top) of an extractive distillation column having a separation efficiency of 60 theoretical stages at a rate of 3870 g/h. 200 g/h of a 0.3 % by weight aqueous solution of hydrazine hydrate was fed to stage 15 (counted from top) of the extractive distillation column. A purified propene oxide, containing 5 ppm methanol and 50 ppm acetaldehyde was obtained as overhead product of the column.

The bottom product of the extractive distillation column was combined with the bottom product obtained from the pre-separation column and subjected to continuous hydrogenation in a trickle-bed reactor. The reactor had an interior volume of 2 I and was filled with an extruded nickel hydrogenation catalyst comprising 50 % by weight Nickel. The hydrogenation was performed at 90 °C and 1.5 MPa at a hydrogen flow rate of 30 NI/h. The resulting hydrogenated solvent mixture comprised on average 49 ppm trimethylamine, 97 ppm ethyldimethylamine and 48 ppm diethylmethylamine, as determined by GC analysis.

The hydrogenated solvent mixture was depressurized and fed to stage 14 (counted from top) of a first methanol distillation column having 20 theoretical stages operated continuously at 0.5 MPa. The hydrogenated solvent mixture was fed at a rate of 7 kg/h. 2 kg/h of a first recovered methanol stream containing 96 % by weight methanol and 4 % by weight water was obtained as overhead product. The bottoms product was fed to a second methanol distillation column having 30 theoretical stages operated continuously at 1.0 MPa. 4.5 kg/h of a second recovered methanol stream containing 96 % by weight methanol and 4 % by weight water was obtained as overhead product. 10 % by weight of aqueous sulfuric acid was added to the hydrogenated solvent mixture before it was fed to the first methanol distillation column at a rate providing an apparent pH of 2.21 in the bottoms product of the second methanol distillation. The first recovered methanol stream had a total nitrogen content of 18 ppm, the second recovered methanol stream of 34 ppm, where the total nitrogen content was determined with a nitrogen analyzer by combustion of a sample and measurement of the molecular nitrogen formed.

The overhead products of the first and the second methanol distillation column were combined and the combined recovered methanol stream was passed through one of two parallel ion exchange columns each containing 3.5 I of DOWEX™ Marathon™ C ion exchange resin. When the apparent pH after the ion exchanger increased to a value above 6.0, the recovered methanol stream was passed to the other ion exchange column and the used ion exchange column was regenerated. The average cycle time of the ion exchangers was 190 h.

### Example 4, epoxidation of propene with extractive distillation of crude propene oxide adding 1,2-diaminoethane as reactive compound

Example 3 was repeated, but 200 g/h of a 2.1 wt.-% solution of ethylenediamine was fed to the extraction column instead of the aqueous solution of hydrazine hydrate. The 10 % by weight of aqueous sulfuric acid was added to the hydrogenated solvent mixture to provide an apparent pH of 2.15 in the bottoms product of the second methanol distillation.

A purified propene oxide, containing less than 5 ppm methanol and less than 50 ppm acetaldehyde was obtained as overhead product of the extractive distillation column. The hydrogenated solvent mixture comprised on average less than 1 ppm trimethylamine, less than 10 ppm ethyldimethylamine and less than 10 ppm diethylmethylamine. The first recovered methanol stream had a total nitrogen content of 2.5 ppm, the second recovered methanol stream of 9.5 ppm. The cycle time of the ion exchangers was 650 h.

Examples 3 and 4 demonstrate that the use of a diaminoalkane instead of hydrazine as reactive compound for acetaldehyde removal leads to reduced formation of volatile amines in the hydrogenation step and to a reduced nitrogen content in the recovered methanol, as well as to an increased time on stream for the ion exchanger used for treating the recovered methanol.

### Examples 5, 6 and 7, epoxidation of propene with extractive distillation of crude propene oxide adding 1,2-diaminoethane as reactive compound

Example 4 was repeated, but the amount of aqueous sulfuric acid added to the hydrogenated solvent mixture was reduced, maintaining an apparent pH of 2.58, 3.03 or 3.13 in the bottoms product of the second methanol distillation. Table 2 summarizes the apparent pH maintained and the total nitrogen content in the first and second recovered methanol stream.

**Table 2**

| apparent pH maintained | nitrogen in first recovered methanol stream in ppm | nitrogen in second recovered methanol stream in ppm |
|---|---|---|
| 2.58 | 5 | 15 |
| 3.03 | 11 | 23 |
| 3.13 | 9 | 22 |

Examples 3 and 4 demonstrate that the use of a diaminoalkane instead of hydrazine allows to reduce the amount of acid added for preventing carryover of ammonia and volatile amines to the recovered methanol, which in turn reduces acid corrosion of the distillation equipment.

## Claims

1. A process for the epoxidation of propene comprising the steps
a) reacting propene with hydrogen peroxide in the presence of a methanol solvent and a titanium zeolite epoxidation catalyst to provide a reaction mixture,
b) separating a crude propene oxide, comprising from 15 to 97 % by weight propene oxide, from 2 to 84 % by weight methanol and acetaldehyde, and a solvent mixture, comprising methanol, water and peroxides, from the reaction mixture of step a),
c) subjecting the crude propene oxide separated in step b) to an extractive distillation in an extractive distillation column, using water as extraction solvent to provide purified propene oxide as an overhead product and a bottoms product comprising water and methanol,
d) subjecting the solvent mixture separated in step b) and the bottoms product of step c) to catalytic hydrogenation to form a hydrogenated solvent mixture,
e) separating the hydrogenated solvent mixture in at least one distillation stage to provide a recovered methanol as an overhead product, adding an acid to the hydrogenated solvent mixture before it is fed to the distillation or to at least one of the distillation stages and
f) recycling the recovered methanol of step e) to step a),
wherein in step c) a reactive compound selected from diaminoalkanes having from 2 to 6 carbon atoms is added to the extractive distillation either with a feed stream to the extractive distillation column or directly to the extractive distillation column at a feed point above the feed point for the crude propene oxide.

2. The process of claim 1, wherein the reactive compound is selected from the group consisting of 1,2-diaminoethane, 1,2-diaminopropane and 1,3-diaminopropane.

3. The process of claim 2, wherein the reactive compound is 1,2-diaminoethane.

4. The process of any one of claims 1 to 3, wherein the reactive compound is fed to the extractive distillation column admixed with the extraction solvent.

5. The process of any one of claims 1 to 4, wherein the solvent mixture separated in step b) and the bottoms product of step c) are combined before subjecting them to catalytic hydrogenation in step d).

6. The process of any one of claims 1 to 5, wherein the crude propene oxide comprises from 4 to 4000 ppm by weight acetaldehyde.

7. The process of any one of claims 1 to 6, wherein the molar ratio of the reactive compound to acetaldehyde is from 0.5 to 10.

8. The process of any one of claims 1 to 7, wherein the mass ratio of the extraction solvent relative to the amount of methanol contained in the crude propene oxide fed is from 0.01 to 1.

9. The process of any one of claims 1 to 8, wherein in step e) acid is added in an amount providing an apparent pH of from 1.8 to 5.0 in the bottoms product remaining after recovery of methanol.

10. The process of any one of claims 1 to 9, wherein sulfuric acid is added in step e).

11. The process of any one of claims 1 to 10, wherein step f) comprises passing the recovered methanol of step e) through a bed of an acidic ion exchanger before recycling it to step a).

12. The process of any one of claims 1 to 11, wherein in step a) ammonia is added with a weight ratio of ammonia to the initial amount of hydrogen peroxide of from 0.0001 to 0.003.

13. The process of any one of claims 1 to 12, wherein steps a) to f) are carried out continuously.

## Patentansprüche

1. Verfahren zur Epoxidierung von Propen, umfassend die Schritte
a) Umsetzen von Propen mit Wasserstoffperoxid in Gegenwart eines Methanol-Lösungsmittels und eines Titanzeolith-Epoxidierungskatalysators zur Bereitstellung einer Reaktionsmischung,
b) Abtrennen eines rohen Propenoxids, das 15 bis 97 Gew.-% Propenoxid, 2 bis 84 Gew.-% Methanol und Acetaldehyd umfasst, und eines Lösungsmittelgemischs, das Methanol, Wasser und Peroxide umfasst, aus der Reaktionsmischung aus Schritt a),
c) Extraktivdestillation des in Schritt b) abgetrennten rohen Propenoxids in einer Extraktivdestillationskolonne unter Verwendung von Wasser als Extraktionslösungsmittel zur Bereitstellung von gereinigtem Propenoxid als Kopfprodukt und eines Sumpfprodukts, das Wasser und Methanol umfasst,
d) katalytische Hydrierung des in Schritt b) abgetrennten Lösungsmittelgemischs und des Sumpfprodukts aus Schritt c) unter Bildung eines hydrierten Lösungsmittelgemischs,
e) Trennen des hydrierten Lösungsmittelgemischs in mindestens einer Destillationsstufe zur Bereitstellung von zurückgewonnenem Methanol als Kopfprodukt, wobei dem hydrierten Lösungsmittelgemisch eine Säure zugesetzt wird bevor es der Destillation oder mindestens einer der Destillationsstufen zugeführt wird, und
f) Zurückführen des zurückgewonnenen Methanols aus Schritt e) in Schritt a),
wobei in Schritt c) eine aus Diaminoalkanen mit 2 bis 6 Kohlenstoffatomen ausgewählte reaktive Verbindung in die Extraktivdestillation zugegeben wird, und zwar entweder mit einem Einsatzstrom in die Extraktivdestillationskolonne oder direkt in die Extraktivdestillationskolonne an einem Einspeisepunkt oberhalb des Einspeisepunkts für das rohe Propenoxid.

2. Verfahren nach Anspruch 1, bei dem die reaktive Verbindung ausgewählt ist aus der Gruppe bestehend aus 1,2-Diaminoethan, 1,2-Diaminopropan und 1,3-Diaminopropan.

3. Verfahren nach Anspruch 2, bei dem es sich bei der reaktiven Verbindung um 1,2-Diaminoethan handelt.

4. Verfahren nach einem der Ansprüche 1 bis 3, bei dem die reaktive Verbindung mit dem Extraktionslösungsmittel gemischt der Extraktivdestillationskolonne zugeführt wird.

5. Verfahren nach einem der Ansprüche 1 bis 4, bei dem das in Schritt b) abgetrennte Lösungsmittelgemisch und das Sumpfprodukt aus Schritt c) vereinigt werden, bevor sie der katalytischen Hydrierung in Schritt d) unterworfen werden.

6. Verfahren nach einem der Ansprüche 1 bis 5, bei dem das rohe Propenoxid 4 bis 4000 Gew.-ppm Acetaldehyd umfasst.

7. Verfahren nach einem der Ansprüche 1 bis 6, bei dem das Molverhältnis von reaktiver Verbindung zu Acetaldehyd 0,5 bis 10 beträgt.

8. Verfahren nach einem der Ansprüche 1 bis 7, bei dem das Massenverhältnis von Extraktionslösungsmittel zur in dem zugeführten rohen Propenoxid enthaltenen Methanolmenge 0,01 bis 1 beträgt.

9. Verfahren nach einem der Ansprüche 1 bis 8, bei dem in Schritt e) Säure in einer Menge zugegeben wird, die in dem nach Rückgewinnung von Methanol verbleibenden Sumpfprodukt einen scheinbaren pH-Wert von 1,8 bis 5,0 ergibt.

10. Verfahren nach einem der Ansprüche 1 bis 9, bei dem in Schritt e) Schwefelsäure zugegeben wird.

11. Verfahren nach einem der Ansprüche 1 bis 10, bei dem in Schritt f) das zurückgewonnene Ethanol aus Schritt e) durch eine Schüttung eines sauren Ionenaustauschers geleitet wird, bevor es in Schritt a) zurückgeführt wird.

12. Verfahren nach einem der Ansprüche 1 bis 11, bei dem in Schritt a) Ammoniak in einem Gewichtsverhältnis von Ammoniak zur Anfangsmenge an Wasserstoffperoxid von 0,0001 bis 0,003 zugegeben wird.

13. Verfahren nach einem der Ansprüche 1 bis 12, bei dem die Schritte a) bis f) kontinuierlich durchgeführt werden.

## Revendications

1. Procédé d'époxydation de propène comprenant les étapes de
a) réaction de propène avec du peroxyde d'hydrogène en présence d'un solvant méthanolique et d'un catalyseur d'époxydation de zéolite au titane pour obtenir un mélange de réaction,
b) séparation d'un oxyde de propène brut, comprenant de 15 à 97 % en poids d'oxyde de propène, de 2 à 84 % en poids de méthanol et d'acétaldéhyde, et un mélange de solvants, comprenant du méthanol, de l'eau et des peroxydes, à partir du mélange de réaction de l'étape a),
c) soumission de l'oxyde de propène brut séparé dans l'étape b) à une distillation extractive dans une colonne de distillation extractive, en utilisant de l'eau en tant que solvant d'extraction pour produire de l'oxyde de propène purifié en tant que produit de tête de distillation et un produit de queue comprenant de l'eau et du méthanol,
d) soumission du mélange de solvants séparé dans l'étape b) et du produit de queue de l'étape c) à une hydrogénation catalytique pour former un mélange de solvants hydrogéné,
e) séparation du mélange de solvants hydrogéné dans au moins un étage de distillation pour obtenir un méthanol récupéré en tant que produit de tête de distillation, en ajoutant un acide au mélange de solvants hydrogéné avant qu'il soit alimenté dans l'étage de distillation ou au moins un des étages de distillation et
f) recyclage du méthanol récupéré de l'étape e) à l'étape a),
dans lequel, dans l'étape c), un composé réactif choisi parmi des diaminoalcanes ayant de 2 à 6 atomes de carbone est ajouté à la distillation extractive avec un flux d'alimentation dans la colonne de distillation extractive ou directement dans la colonne de distillation extractive à un point d'alimentation au-dessus du point d'alimentation pour l'oxyde de propène brut.

2. Procédé selon la revendication 1, dans lequel le composé réactif est choisi dans le groupe constitué des 1,2-diaminoéthane, 1,2-diaminopropane et 1,3-diaminopropane.

3. Procédé selon la revendication 2, dans lequel le composé réactif est le 1,2-diaminoéthane.

4. Procédé selon l'une quelconque des revendications 1 à 3, dans lequel le composé réactif est alimenté dans la colonne de distillation extractive mélangé avec le solvant d'extraction.

5. Procédé selon l'une quelconque des revendications 1 à 4, dans lequel le mélange de solvants séparé dans l'étape b) et le produit de queue de l'étape c) sont combinés avant la soumission de ceux-ci à une hydrogénation catalytique dans l'étape d).

6. Procédé selon l'une quelconque des revendications 1 à 5, dans lequel l'oxyde de propène brut comprend de 4 à 4000 ppm en poids d'acétaldéhyde.

7. Procédé selon l'une quelconque des revendications 1 à 6, dans lequel le rapport molaire du composé réactif à l'acétaldéhyde est de 0,5 à 10.

8. Procédé selon l'une quelconque des revendications 1 à 7, dans lequel le rapport en masse du solvant d'extraction par rapport à la quantité de méthanol contenue dans l'oxyde de propène brut alimenté est de 0,01 à 1.

9. Procédé selon l'une quelconque des revendications 1 à 8, dans lequel, dans l'étape e), un acide est ajouté en une quantité conférant un pH apparent de 1,8 à 5,0 dans le produit de de queue restant après la récupération de méthanol.

10. Procédé selon l'une quelconque des revendications 1 à 9, dans lequel l'acide sulfurique est ajouté dans l'étape e).

11. Procédé selon l'une quelconque des revendications 1 à 10, dans lequel l'étape f) comprend le passage du méthanol récupéré de l'étape e) à travers un lit d'un échangeur d'ions acide avant son recyclage dans l'étape a).

12. Procédé selon l'une quelconque des revendications 1 à 11, dans lequel, dans l'étape a), de l'ammoniac est ajouté avec un rapport en poids d'ammoniac à la quantité initiale de peroxyde d'hydrogène de 0,0001 à 0,003.

13. Procédé selon l'une quelconque des revendications 1 à 12, dans lequel les étapes a) à f) sont conduites en continu.
